# EUROPEAN PATENT SPECIFICATION

(11) **EP 3 452 051 B1**
(45) Date of publication and mention of the grant of the patent: **01.06.2022**
(21) Application number: 17792541.9
(22) Date of filing: 05.05.2017
(51) Int. Cl.: A61K 31/702, A61P 1/14, A23L 33/125, A23C 9/20

(54) **COMPOSITION COMPRISING HMOS FOR USE IN THE TREATMENT OF MAST CELL MEDIATED VISCERAL HYPERSENSITIVITY AND/OR PAIN**
ZUSAMMENSETZUNG MIT HMOS ZUR VERWENDUNG IN DER BEHANDLUNG VON MASTZELLENVERMITTELTER VISZERALER ÜBEREMPFINDLICHKEIT UND/ODER SCHMERZEN
COMPOSITION COMPRENANT DU HMOS DESTINÉE À ÊTRE UTILISÉE DANS LE TRAITEMENT DE L'HYPERSENSIBILITÉ ET/OU DE LA DOULEUR VISCÉRALE MÉDIÉE PAR LES MASTOCYTES

(30) Priority: 05.05.2016 US 201615147115
(43) Date of publication of application: 13.03.2019
(73) Proprietor: Glycom A/S, 2970 Hørsholm (DK)
(72) Inventor: VIGSNÆS, Louise Kristine, 2400 Copenhagen NV (DK); MCCONNELL, Bruce, 1814 La Tour de Peilz (CH); ELISON, Emma, 21759 Malmö (SE)
(74) Representative: Inspicos P/S
(86) International application number: PCT/DK2017/050146
(87) International publication number: WO 2017/190755

(56) References cited:
- EP-A1- 2 455 387
- WO-A1-2010/105207
- WO-A1-2016/066175
- WO-A1-2017/071715
- WO-A1-2019/121929
- US-A1- 2012 171 165
- US-A1- 2013 195 803
- US-A1- 2015 004 147
- US-A1- 2016 243 139
- BARBARA ET AL: "Activated mast cells in proximity to colonic nerves correlate with abdominal pain in irritable bowel syndrome", GASTROENTEROLOGY : OFFICIAL PUBLICATION OF THE AMERICAN GASTROENTEROLOGICAL ASSOCIATION, WILLIAMS & WILKINS, US, vol. 126, no. 3, 1 March 2004 (2004-03-01) , pages 693-702, XP005314478, ISSN: 0016-5085, DOI: 10.1053/J.GASTRO.2003.11.055
- IRIBARREN CRISTINA ET AL: "1142 - The Effects of Human Milk Oligosaccharides on Bifidobacteria and Gastrointestinal Symptoms in Irritable Bowel Syndrome Patients: A Parallel, Double Blind, Randomized, Placebo-Controlled Trial", GASTROENTEROLOGY : OFFICIAL PUBLICATION OF THE AMERICAN GASTROENTEROLOGICAL ASSOCIATION, vol. 156, no. 6, 2019, XP085677430, ISSN: 0016-5085, DOI: 10.1016/S0016-5085(19)37409-8

## Description

### FIELD OF THE INVENTION

This invention relates generally to compositions and methods for preventing or treating mast cell mediated visceral pain.

### BACKGROUND TO THE INVENTION

Visceral hypersensitivity and pain represent a major clinical problem, yet far less is known about their mechanisms compared to somatic pain. Visceral hypersensitivity and pain are defined as pain felt arising from the internal organs (viscera) of the body. There are multiple aetiologies for pain sensed in viscera, including inflammation (acute and chronic), infection, disruption of normal mechanical processes (e.g. gastrointestinal dysmotility) or alterations in nerves. The recent growth in interest in pain originating from internal organs reflects an important paradigm shift in the awareness of the magnitude and impact of visceral pain disorders.

Visceral pain usually has a temporal evolution and clinical features vary in different phases of pathology. 'True visceral pain' arises as a diffuse and poorly defined sensation usually perceived in the midline of the body, at the lower sternum or upper abdomen. In patients, pain from different visceral organs can have differing areas of presentation, e.g. bladder to perineal area, heart to left arm and neck, left ureter to left lower quadrant and loin. This diffuse nature and difficulty in locating visceral pain is due to a low density of visceral sensory innervation and extensive divergence of visceral input within the central nerve system. Visceral pain is therefore perceived more diffusely than noxious cutaneous stimulation with respect to location and timing. Visceral pain is often associated with marked autonomic phenomena, including pallor, profuse sweating, nausea, gastrointestinal disturbances and changes in body temperature, blood pressure and heart rate (Sikandar et al. Curr. Opin. Support. Palliat. Care 6, 17 (2012)).

The "brain-gut axis" is a theoretical model depicting bidirectional neural pathways linking cognitive, emotional and autonomic centres in the brain to neuroendocrine centres, the enteric nervous system and the immune system. The gastrointestinal tract possesses both the largest neural network outside the brain and the most extensive immune system, which brings about the opportunity for crosstalk between neurons and immune cells, including mast cells. Many studies have confirmed that endings of postganglionic sympathetic, peptidergic and vagal fibres, and enteric neurons are in close proximity to mast cells. It has been estimated that 70% of intestinal mucosal mast cells are in direct contact with nerves. Due to their location mast cells play an important role in the regulation of gastrointestinal visceral hypersensitivity and vascular permeability, and an alteration in the mast cells - nerve axis can contribute to autonomic dysregulation of the gut and associated pain and perceptual changes in visceral disorders. Several studies have noted an increased number of mast cells in the mucosa of patients with gastrointestinal diseases such as irritable bowel syndrome, mastocytic enterocolitis, and systemic mastocytosis (Buhner et al. Biochim. Biophys. Acta 1822, 85 (2012)).

The functional mast cell-neuronal units consist of 2 pathways: the nerve to mast cells signalling and the mast cells to nerve signalling. One of the important mediators in this process is mast cell tryptase, which activate PAR2 present on sensory afferents. PAR2 plays a crucial role in sensitizing afferent neurons and also causes the release of substance P and CGRP. This system acts to amplify the inflammatory response with the gastrointestinal tract and leads to increased motility and secretion as part of the ENS response to mast cell degranulation. Mast cells hyperplasia and activation can lead to abnormal gastrointestinal sensitivity, motility, and secretion, which in turn contribute to the hallmark symptoms found in functional gastrointestinal disorders including abdominal pain and/or discomfort, bloating, and abnormal bowel function (diarrhoea and/or constipation) (Zhang et al. J. Neurogastroenterol. Motil. 22, 181 (2016)).

Beside the mast cells - nerve axis, emerging data show that there is an interaction between the intestinal microbiota and pathways mediating visceral pain. The absence of gastrointestinal bacteria, such as which occurs in germ free mice, is associated with reduced perception of pain following different inflammatory stimuli. Furthermore, modulation of the intestinal microbiota by administration of various probiotics or prebiotics also has been shown to alter pain responses.

Visceral pain is a defining criteria for irritable bowel syndrome (IBS). IBS is a clinically heterogeneous disorder of human patients, particularly adult, with chronic symptoms such as abdominal pain, abdominal discomfort, abdominal bloating, fatigue, and changes in bowel movement patterns, such as patterns of loose or more frequent bowel movements, diarrhoea and constipation. Routine clinical tests on patients typically show no abnormalities, although their bowels may be more sensitive to certain stimuli, such as balloon insufflation testing. The worldwide prevalence of IBS is about 10-20 % but may be higher in certain countries (Longstreth et al. Gastroenterology 130, 1480 (2006)). The causes of IBS are unknown but disruptions of the brain-gut axis, acute gastrointestinal infections, small intestinal bacterial overgrowths, antibiotic usages and dysbiosis are thought to be important risk factors (Kim et al. Dig. Dis. Sci. 57, 3213 (2012)). Other risk factors are young age, prolonged fever, anxiety, and depression. Chronic low-grade inflammation commonly occurs in IBS patients, but there are otherwise little or no observable clinical manifestations.

Diagnosis of IBS is difficult. No biomarker-based tests can be performed to diagnose IBS. Diagnosis generally involves excluding conditions that produce IBS-like symptoms and then following a procedure to categorise a patient's symptoms. Ruling out parasitic infections, lactose intolerance, and celiac disease is recommended for all patients before a diagnosis of IBS is made. Once diagnosed, patients are usually classified in accordance with the Rome IV criteria into four symptom subtypes based on stool consistency: diarrhoea-predominant (IBS-D), constipation-predominant (IBS-C), mixed subtype (IBS-M or IBS-A) with alternating episodes of both diarrhoea and constipation, and unsubtyped IBS (IBS-U).

There is no cure for IBS and current treatments focus on attempting to relieve symptoms; including visceral pain but current pain treatments have limited efficacy. Treatments take various forms such as dietary adjustments, medication, and psychological interventions. Patient education and good doctor-patient relationships are also important. However, most treatment is unsatisfactory and most patients continue to experience chronic pain, fatigue, and other symptoms. While IBS has no direct effect on life expectancy, its high prevalence and significant effects on quality of life make it a condition with a high social cost. The general hopelessness associated with IBS is a source of frustration for both patients and health care practitioners treating them.

Current research has implicated the gastrointestinal microbiota, the brain-gut axis and the mast cells in the pathophysiology of IBS. The human gastrointestinal microbiota includes at least 1000 species of bacteria, and about 10¹⁴ individual bacterial cells from about 160 different species inhabit each individual's intestine (Qin et al. Nature 464, 59 (2010)). It is believed that an individual's genetic make-up and acquired immunity, as well as environmental factors, influence their gastrointestinal microbiota. The microbiota in turn shape the individual's immunity and physiology within the gastrointestinal system. It is also believed that a healthy individual maintains a symbiotic relationship with the microbiota colonizing his/her intestines, while an individual with IBS has an imbalance in this microbiota-host interaction.

Mast cells are believed to play an important role in the pathogenesis of IBS. Increased mast cell infiltration and activation in distal gut segments are associated with symptom onset and severity of IBS. These cells are also implicated in the elevated response of visceral afferent nerves to mucosal stimulus in IBS patients. Mast cell hyperplasia is commonly observed following infection by bacteria in both post-infectious IBS and non-post-infectious IBS.

WO 2016/066175 relates to synthetic compositions containing one or more human milk oligosaccharides for use in treating irritable bowel syndrome and symptoms relating to irritable bowel syndrome.

US 2012/0171165 discloses nutritional compositions including human milk oligosaccharides that can be administered to individuals for improving gastrointestinal function and tolerance, as well as the growth of beneficial bacteria.

Barbara et al. (Gastroenterology 126, 693 (2004)) suggest that colonic mast cell infiltration and mediator release in proximity to mucosal innervation may contribute to abdominal pain perception in IBS patients.

Therefore, there is a need for a safe and effective intervention for the treatment of visceral pain disorders mitigated by mast cells.

### SUMMARY OF THE INVENTION

This invention provides synthetic compositions comprising one or more human milk oligosaccharides (HMOs), that can be advantageously used for the prophylaxis or treatment of mast cell mediated visceral hypersensitivity and/or mast cell mediated visceral pain in a human, in particular a non-infant human individual.

Accordingly:
- a first aspect of this invention relates to a human milk oligosaccharide for use in the prophylaxis or treatment of mast cell mediated visceral hypersensitivity and/or mast cell mediated visceral pain in a human;
- a second aspect of this invention relates to a synthetic composition for use in the prophylaxis or treatment of mast cell mediated visceral hypersensitivity and/or mast cell mediated visceral pain in a human, the composition comprising an effective amount of one or more human milk oligosaccharides.

In one embodiment, the human is an IBS patient.

Preferably the amount of a human milk oligosaccharide is effective to (i) increase the abundance, particularly the relative abundance, of bifidobacteria, and/or (ii) improve the gut barrier function of the human. More preferably the one or more HMOS are administered to a non-infant human in two steps:
(a) in a first step during an initial treatment period of about 14 days to increase the relative abundance of bifidobacteria of the phylogenetic *Bifidobacterium adolescentis* group; and
(b) in a second step, during an additional period of treatment of 1 or more days following the initial treatment period, to increase the relative abundance of *Bifidobacterium longum* and/or

### Bifidobacterium bifidum,

in the microbiota in the gastro-intestinal tract of said non-infant human.

The patient may have intestinal dysbiosis and/or an impaired mucosal barrier.

Preferably, the human milk oligosaccharide is 2'-FL, 3-FL, DFL, LNnT, LNT, 3'-SL, 6'-SL or LNFP-I or a mixture thereof. For example, the composition can comprise a mixture of a fucosylated HMO such as 2'-FL and/or DFL and a non-fucosylated neutral HMO such as LNnT or LNT, or both. In one preferred embodiment, the HMO is a mixture of 2'-FL and LNnT and/or LNT. In this embodiment, the 2'-FL and LNnT/LNT may be present in a mass ratio of about 5:1 to 1:1; more preferably about 4:1 to 2:1. In another preferred embodiment, the human milk oligosaccharide is a mixture of 2'-FL and/or DFL and LNnT and/or LNT. In this embodiment, the 2'-FL/DFL and LNnT/LNT may be present in a mass ratio of about 5:1 to 1:1; more preferably about 4:1 to 2:1.

The synthetic composition can be a nutritional or pharmaceutical composition. Preferably, synthetic composition of the invention is administered daily. Furthermore, the synthetic composition is preferably administered for a period of at least one month, such as at least 2 months or for a longer period of time, for example chronically on an ongoing basis.

The synthetic composition may be administered to the human or patient as a daily dose of about 3 g to about 15 g such as from about 3 g to about 10 g of HMOs. The patient can be administered a higher amount, preferably 5 g to 10 g per day, of the HMOs for up to 8 weeks, followed by a lower amount, preferably 3 g to 5 g per day, for a period of time afterwards, which period can be from 1 week to 1 month or even longer.

### DETAILED DESCRIPTION OF THE INVENTION

It has been surprisingly found that human milk oligosaccharides (HMOs) are able to modulate the gut microbiota, stabilize mast cells and hereby prevent symptoms in patients suffering from visceral pain. HMOs preferentially increase the abundance of beneficial bacteria, such as bifidobacteria, and regulate immune responses causing a decrease in the degranulation and activation of mast cells. Furthermore, HMOs act to repair damage in the mucosal barrier and act on neuronally dependent gut migrating motor complexes to address disorders of gut motility and possibly have beneficial effects on the central nervous systems of patients. As an outcome, visceral hypersensitivity and/or mast cell mediated visceral pain is reduced.

The term "oral administration" preferably means any conventional form for the oral delivery of a composition to a patient that causes the deposition of the composition in the gastrointestinal tract (including the stomach) of the patient. Accordingly, oral administration includes swallowing of composition by the patient, enteral feeding through a naso-gastric tube, and the like.

The term "effective amount" preferably means an amount of a composition that provides a human milk monosaccharide or human milk oligosaccharide in a sufficient amount to render a desired treatment outcome in a patient. An effective amount can be administered in one or more doses to the patient to achieve the desired treatment outcome.

The term "human milk oligosaccharide" or "HMO" preferably means a complex carbohydrate found in human breast milk that can be in acidic or neutral form. More than about 200 different HMO structures are known to exist in human breast milk (Urashima et al.: Milk Oligosaccharides, Nova Biomedical Books, New York, 2011). HMOs can be backbone, fucosylated and sialylated oligosaccharides. Backbone HMOs consists of Glu, Gal and GIcNAc and are devoid of Fuc and sialic acid. Examples of backbone HMOs include lacto-N-tetraose (LNT), lacto-N-neotetraose (LNnT), lacto-N-neohexaose (LNnH) and lacto-N-hexaose (LNH). Fucosyl HMOs are fucosylated lactoses or fucosylated backbone HMOs such as 2'-fucosyllactose (2'-FL), lacto-N-fucopentaose I (LNFP-I), lacto-N-difucohexaose I (LNDFH-I), 3-fucosyllactose (3-FL), difucosyllactose (DFL), lacto-N-fucopentaose III (LNFP-III), fucosyl-para-lacto-N-neohexaose (F-pLNnH), lacto-N-difucohexaose I (LNDFH-I), fucosyl-lacto-N-hexaose II (FLNH-II), lacto-N-fucopentaose V (LNFP-V), lacto-N-difucohexaose II (LNDFH-II), fucosyl-lacto-N-hexaose I (FLNH-I), fucosyl-lacto-N-hexaose III (FLNH-III) and fucosyl-para-lacto-N-neohexaose (F-pLNnH). Sialyl HMOs are sialylated lactoses or sialylated backbone HMOs such as 3',6-disialyllacto-N-tetraose (DSLNT), 6'-sialyllactose (6'-SL), 3'-sialyllactose (3'-SL), 6'-sialyllacto-N-neotetraose (LST c), 3'-sialyllacto-N-tetraose (LST a) and 6-sialyllacto-N-tetraose (LST b). HMOs containing both sialyl and fucosyl groups may be considered to belong to either of the latter two groups. Examples for sialyl and fucosyl HMOs include disialyl-fucosyl-lacto-N-hexaose II (DSFLNH-II), fucosyl-sialyl-lacto-N-neohexaose I (FSLNnH-I), fucosyl-sialyl-lacto-N-hexaose I (FSLNH-I) and 3-fucosyl-3'-sialyllactose (FSL). In context of the present invention, lactose is not included in the group of HMOs.

The terms "microbiota", "microflora" and "microbiome" preferably mean a community of living microorganisms that typically inhabits a bodily organ or part. The most dominant members of the gastrointestinal microbiota include microorganisms of the phyla of *Firmicutes, Bacteroidetes, Actinobacteria, Proteobacteria, Synergistetes, Verrucomicrobia, Fusobacteria,* and *Euryarchaeota;* at genus level the microorganisms of *Bacteroides, Faecalibacterium, Bifidobacterium, Roseburia, Alistipes, Collinsella, Blautia, Coprococcus, Ruminococcus, Eubacterium* and *Dorea;* and at species level microorganisms of *Bacteroides uniformis, Alistipes putredinis, Parabacteroides merdoe, Ruminococcus bromii, Doreo longicatena, Bacteroides caccae, Bacteroides thetaiotaomicron, Eubacterium hallii, Ruminococcus torques, Faecalibacterium prausnitzii, Ruminococcus lactaris, Collinsella aerofaciens, Dorea formicigenerans, Bacteroides vulgatus* and *Roseburia intestinalis.* In some instances, the gastrointestinal microbiota includes the mucosa-associated microbiota, which is located in or attached to the mucus layer covering the epithelium of the gastrointestinal tract, and luminal-associated microbiota, which is found in the lumen of the gastrointestinal tract.

The terms "irritable bowel syndrome" and "IBS" preferably mean a group of functional bowel disorders of humans, particularly adults, characterized by one or more chronic symptoms including abdominal pain, abdominal discomfort, abdominal bloating, fatigue, and changes in bowel movement patterns, such as patterns of loose or more frequent bowel movements, diarrhoea and constipation, typically in the absence of any apparent structural abnormality. There are at least three forms of IBS, depending on which symptom predominates: (1) diarrhoea-predominant (IBS-D); (2) constipation-predominant (IBS-C); and (3) IBS with alternating stool pattern (IBS-A or IBS-M). There are also various clinical subtypes of IBS, such as post-infectious IBS (IBS-PI) or unsubtyped IBS (IBS-U).

The term "bifidobacteria" means a member of the *Bifidobacterium* genus commonly found in the human gastro-intestinal tract. Examples of bifidobacteria are: *Bifidobacterium longum, Bifidobacterium bifidum,* and members of the phylogenetic *Bifidobacterium adolescentis* group. Bifidobacteria of the phylogenetic *Bifidobacterium adolescentis* group are, for example, *Bifidobacterium pseudocatenulatum* and/or *Bifidobacterium adolescentis.*

The term "synthetic composition" means a composition which is artificially prepared and preferably means a composition containing at least one compound that is produced *ex vivo* chemically and/or biologically, e.g. by means of chemical reaction, enzymatic reaction or recombinantly. In some embodiments a synthetic composition may be, but preferably is not, identical with a naturally occurring composition. The synthetic composition typically comprises one or more HMOs that are capable of preferentially increasing the abundance of bifidobacteria. In some embodiments, the synthetic composition may comprise one or more compounds or components other than HMOs that may have an effect on bifidobacteria of a human subject microbiota *in vivo,* e.g. non-digestible oligosaccharides or prebiotics. Also in some embodiments, the synthetic compositions may comprise one or more nutritionally or pharmaceutically active components which do not affect adversely the efficacy of the above mentioned compounds. Some non-limiting embodiments of a synthetic composition of the invention are also described below. The synthetic composition preferably comprises an effective amount of one or more HMOs.

The term "effective amount" preferably means that the amount of a human milk oligosaccharide is effective to (i) increase the abundance, particularly the relative abundance, of bifidobacteria, and/or (ii) improve the gut barrier function of the human.

The effective amount of HMO(s) may vary depending on compound selected from the groups of HMOs described herein. The effective amount may be in the range from about 1 to about 10 g or more of HMOs per administration dose; in some preferred embodiments, the effective amounts are from about 3 to about 10 g per administration dose.

The term "relative abundance of bifidobacteria" means the abundance of bifidobacteria relative to other genus in the microbiota of the gastro-intestinal tract.

The term "relative growth of bifidobacteria" means the growth of bifidobacteria relative to other genus in the microbiota in the gastro-intestinal tract.

The term "non-infant human" or "non-infant" means in the present context a human of 3 years of age and older. A non-infant human can be a child, a teenager, an adult or an elderly.

The term "enteral administration" means any conventional form for delivery of a composition to a human that causes the deposition of the composition in the gastrointestinal tract (including the stomach). Methods of enteral administration include feeding through a naso-gastric tube or jejunum tube, oral, sublingual and rectal.

The term "oral administration" means any conventional form for the delivery of a composition to a human through the mouth. Accordingly, oral administration is a form of enteral administration.

The term "prophylaxis" means a treatment given or an action taken to prevent a disease in the patient, which disease is associated with or triggered by mast cell mediated visceral hypersensitivity and/or mast cell mediated visceral pain.

The term "initial period" of treatment with an HMO or an HMO mixture is about 14 days from the start of the treatment; "additional period" of treatment means 1 or more days following the initial period of treatment.

The term "visceral hypersensitivity" preferably refers to an increased intensity of sensation of stimuli of visceral organs of the body.

The "visceral organ" is an organ of the digestive, respiratory, urogenital and endocrine systems as well as the spleen, the heart and great vessels. In particular, a person who experiences visceral hypersensitivity may have a lowered threshold for visceral pain, such as abdominal pain and discomfort in response to pressure, stimulation or distension within the abdomen.

The term "visceral pain" preferably refers to a distressing feeling arising from the visceral organs of the body.

The term "about" in the present context means up to 2.5 % deviation from the corresponded value.

The term "preferably" is used herein to indicate the best mode of invention, but not to limit the scope of invention.

### HMOs for use in the prophylaxis or treatment of mast cell mediated visceral hypersensitivity and/or mast cell mediated visceral pain in a human

HMOs for use in the prophylaxis or treatment of mast cell mediated visceral hypersensitivity and/or mast cell mediated visceral pain in a human may be a single HMO or a mixture of any HMOs suitable for the purpose of the invention. Preferably, the HMO is a fucosylated or a non-fucosylated neutral HMO. More preferably, the HMOs for use in the prophylaxis or treatment of mast cell mediated visceral hypersensitivity and/or mast cell mediated visceral pain in a human is a mixture of at least a first HMO and at least a second HMO, wherein the first HMO is a fucosylated neutral HMO and the second HMO is a non-fucosylated neutral HMO. Particularly, the mixture contains a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the mixture contains a fucosylated HMO selected from the list consisting of 2'-FL, 3-FL and DFL, and a non-fucosylated HMO selected from the list consisting of LNT and LNnT; advantageously the mixture comprises 2'-FL and/or DFL and LNnT and/or LNT. In some embodiments, the mixture essentially consists of two neutral HMOs, e.g. a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the mixture essentially consists of a fucosylated HMO selected from the list consisting of 2'-FL, 3-FL and DFL, and a non-fucosylated HMO selected from the list consisting of LNT and LNnT; in one preferred embodiment the mixture essentially consists of 2'-FL and LNnT, in another preferred embodiment the mixture essentially consists of 2'-FL and LNT.

The HMOs can be isolated or enriched by well-known processes from milk(s) secreted by mammals including, but not limited to human, bovine, ovine, porcine, or caprine species. The HMOs can also be produced by well-known processes using microbial fermentation, enzymatic processes, chemical synthesis, or combinations of these technologies. As examples, using chemistry LNnT can be made as described in WO 2011/100980 and WO 2013/044928, LNT can be synthesized as described in WO 2012/155916 and WO 2013/044928, a mixture of LNT and LNnT can be made as described in WO 2013/091660, 2'-FL can be made as described in WO 2010/115934 and WO 2010/115935, 3-FL can be made as described in WO 2013/139344, 6'-SL and salts thereof can be made as described in WO 2010/100979, sialylated oligosaccharides can be made as described in WO 2012/113404 and mixtures of human milk oligosaccharides can be made as described in WO 2012/113405. As examples of enzymatic production, sialylated oligosaccharides can be made as described in WO 2012/007588, fucosylated oligosaccharides can be made as described in WO 2012/127410, and advantageously diversified blends of human milk oligosaccharides can be made as described in WO 2012/156897 and WO 2012/156898. With regard to biotechnological methods, WO 01/04341 and WO 2007/101862 describe how to make core human milk oligosaccharides optionally substituted by fucose or sialic acid using genetically modified *E. coli.*

### Synthetic composition comprising HMOs

The synthetic composition may comprise a single HMO, or a mixture of any HMOs suitable for the purpose of the invention. Preferably, the HMO is a fucosylated or a non-fucosylated neutral HMO. More preferably, the composition comprises a mixture of at least a first HMO and at least a second HMO, wherein the first HMO is a fucosylated neutral HMO and the second HMO is a non-fucosylated neutral HMO. Particularly, the composition may contain a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the composition contains a fucosylated HMO selected from the list consisting of 2'-FL, 3-FL and DFL, and a non-fucosylated HMO selected from the list consisting of LNT and LNnT, advantageously the composition comprises 2'-FL and/or DFL and LNnT and/or LNT. In some embodiments, the composition comprises a mixture essentially consists of two neutral HMOs, e.g. a fucosylated HMO selected from the list consisting of 2'-FL, 3-FL, DFL, LNFP-I, LNFP-II, LNFP-III, LNFP-V, LNDFH-I, LNDFH-II, LNDFH-III, FLNH-I, FLNH-II, FLNnH, FpLNH-I and F-pLNnH II, and a non-fucosylated HMO selected from the list consisting of LNT, LNnT, LNH, LNnH, pLNH and pLNnH. Preferably, the composition comprises a mixture consisting of a fucosylated HMO selected from the list consisting of 2'-FL, 3-FL and DFL, and a non-fucosylated HMO selected from the list consisting of LNT and LNnT; in one preferred embodiment the composition comprises a mixture essentially consisting of 2'-FL and LNnT, in another preferred embodiment the composition comprises a mixture essentially consisting of 2'-FL and LNT.

A synthetic composition of this invention comprising one or more human milk oligosaccharides can take any suitable form. For example, the composition can be in the form of a nutritional composition which contains other macronutrients such as proteins, lipids or other carbohydrates, preferably where the other carbohydrate is lactose and/or a carbohydrate different than an HMO. The synthetic composition can also be a pharmaceutical composition or other unit dose form.

### Nutritional compositions

A nutritional composition of this invention can contain sources of protein, lipids and/or digestible carbohydrates and can be in powdered or liquid forms. The composition can be designed to be the sole source of nutrition or a nutritional supplement. For visceral pain patients, a nutritional supplement is preferred; especially a supplement which can form a meal or snack replacement. Preferably the nutritional composition is lactose-reduced or, better yet, lactose-free. Preferably, the nutritional composition is also free from, or low in amounts of, FODMAP carbohydrates.

Suitable protein sources include milk proteins, soy protein, rice protein, pea protein and oat protein, or mixtures thereof. Milk proteins can be in the form of milk protein concentrates, whey protein or casein, or mixtures of both. Soy, rice, pea and oat protein can be in the form or protein isolated. The protein can be whole protein or hydrolysed protein, either partially hydrolysed or extensively hydrolysed. The protein can provide about 5 % to about 50 %, preferably about 10 % to 30 %, of the energy of the nutritional composition. The protein source preferably is not a source of non-fermentable carbohydrates such as lactose. Therefore, if a milk protein is used as the protein source, the milk protein is preferably lactose-reduced or lactose-free.

The protein source can be a source of glutamine, threonine, cysteine, serine, proline, or a combination of these amino acids. The glutamine source can be a glutamine dipeptide and/or a glutamine enriched protein. Glutamine can be included due to the use of glutamine by enterocytes as an energy source. Threonine, serine and proline are important amino acids for the production of mucin. Mucin coats the GI tract and can reduce permeability. Cysteine is a major precursor of glutathione, which is key for the antioxidant defences of the body.

Suitable digestible carbohydrates include maltodextrin, hydrolysed or modified starch or corn starch, glucose polymers, corn syrup, corn syrup solids, tapioca, sucrose, and glucose, or mixtures thereof. Generally digestible carbohydrates provide about 35 % to about 75 %, preferably about 45 % to 70 %, of the energy of the nutritional composition. Preferably the digestible carbohydrate is free from lactose.

Suitable lipids include rapeseed oil, sunflower seed oil, palm oil, soy oil, milk fat, corn oil and soy lecithin. Long-chain poly unsaturated fatty acids (LC-PUFA), especially omega-3 fatty acids such as docosahexaenoic acid (DHA), can be included in the lipid source because they have anti-inflammatory properties. Suitable sources of LC-PUFA are plant oils, marine plankton oils, fungal oils, and fish oils. The lipid source can also include medium chain triglycerides (MCT). Fractionated coconut oils are a suitable source of medium chain triglycerides. The lipid source preferably provides about 5 % to about 25 % of the energy of the nutritional composition; for example about 10 % to 20 %. The lipid content is preferably reduced because high fat diets can provoke IBS symptoms.

The nutritional composition preferably also includes vitamins and minerals. If the nutritional composition is intended to be a sole source of nutrition, it preferably includes a vitamin and mineral profile, preferably a complete vitamin and mineral profile. The term "complete" in the present context means a vitamin and mineral profile comprising all vitamins and minerals essential for body function, wherein the essential vitamins includes at least 9 vitamins from the exemplary group below, such as 10, 11, 12 or 13, or more, and the essential minerals includes at least 5 minerals from the exemplary group below, such as from 6 to 13 or more. Examples of vitamins include vitamins A, B- complex (such as B1, B2, B6 and B12), C, D, E and K, niacin and acid vitamins such as pantothenic acid and folic acid and biotin. Examples of minerals include calcium, iron, zinc, magnesium, iodine, copper, phosphorus, manganese, potassium, chromium, molybdenum, selenium, nickel, tin, silicon, vanadium and boron.

The nutritional composition can also include a carotenoid such as lutein, lycopene, zeaxanthin, and beta-carotene. The total amount of carotenoid included can vary from about 0.001 µg/ml to about 10 µg/ml. Lutein can be included in an amount of from about 0.001 µg/ml to about 10 µg/ml, preferably from about 0.044 µg/ml to about 5 g/ml of lutein. Lycopene can be included in an amount from about 0.001 µg/ml to about 10 µg/ml, preferably about 0.0185 mg/ml to about 5 g/ml of lycopene. Beta-carotene can comprise from about 0.001 µg/ml to about 10 mg/ml, for example about 0.034 µg/ml to about 5 µg/ml of beta-carotene.

The nutritional composition can also contain various other conventional ingredients such as preservatives, emulsifying agents, thickening agents, buffers, fibres and probiotics, especially probiotics which can help to reduce symptoms in IBS patients (e.g. VSL#3, *B. infantis* 35624, *B. animalis* subsp. *lactis* BB-12, *B. lactis* Bi-07, *L. rhamnosus* GG, *L. rhamnosus* Lc705, *L. plantarum* DSM 9843, *L. plantarum* CECT7484, *L. plantarum* CECT7485, *L. acidophilus* NCFM, *L. fermentum* CECT5716, *B. breve* Bb99, *Propionibacterium freundenreichii* ssp. *Shermanii* JS, *P. acidilactici* CECET7483, *Streptococcus faecium*), antioxidant/anti-inflammatory compounds including tocopherols, carotenoids, ascorbate/vitamin C, ascorbyl palmitate, polyphenols, glutathione, and superoxide dismutase (melon), other bioactive factors (e.g. growth hormones, cytokines, TFG-β), colorants, flavours, and stabilisers, lubricants, and so forth.

The nutritional composition can be in the form of a soluble powder, a liquid concentrate, or a ready-to-use formulation. Various flavours, fibres and other additives can also be present.

The nutritional compositions can be prepared by any commonly used manufacturing techniques for preparing nutritional compositions in solid or liquid form. For example, the composition can be prepared from various feed solutions. A protein-in-fat feed solution can be prepared by heating and mixing the lipid source and then adding an emulsifier (e.g. lecithin), fat soluble vitamins, and at least a portion of the protein source while heating and stirring. A carbohydrate feed solution is also prepared by adding minerals, trace and ultra trace minerals, thickening or suspending agents to water while heating and stirring. The resulting solution is held for 10 minutes with continued heat and agitation before adding carbohydrates (e.g., the HMOs and digestible carbohydrate sources). The resulting feed solutions are then blended together while heating and agitating and the pH adjusted to 6.6-7.0, after which the composition is subjected to high-temperature short-time processing during which the composition is heat treated, emulsified and homogenized, and then allowed to cool. Water soluble vitamins and ascorbic acid are added, the pH is adjusted to the desired range if necessary, flavours are added, and water is added to achieve the desired total solid level.

For a liquid product, the resulting solution can then be aseptically packaged to form an aseptically packaged nutritional composition. In this form, the nutritional composition can be in ready-to-feed or concentrated liquid form. Alternatively, the composition can be spray dried and processed and packaged as a reconstitutable powder.

When the nutritional product is a ready-to-feed nutritional liquid, the total concentration of HMOs in the liquid, by weight of the liquid, is from about 0.02 % to about 2.0 %, including from about 0. 1 % to about 1.5 %, including from about 0.3 % to about 1.0 %. When the nutritional product is a concentrated nutritional liquid, the total concentration of HMOs in the liquid, by weight of the liquid, is from about 0.04 % to about 4.0 %, including from about 0.2 % to about 3.0 %, including from about 0.6 % to about 2.0 %.

### Unit dosage forms

The synthetic composition can also be in a unit dosage form such as a capsule, tablet or sachet. For example, the composition can be in a tablet form comprising the human milk mono- and/or oligosaccharides, and one or more additional components to aid formulation and administration, such as diluents, excipients, antioxidants, lubricants, colorants, binders, disintegrants, and the like.

Suitable diluents, excipients, lubricants, colorants, binders, and disintegrants include polyethylene, polyvinyl chloride, ethyl cellulose, acrylate polymers and their copolymers, hydroxyethyl-cellulose, hydroxypropylmethyl-cellulose (HPMC), sodium carboxymethylcellulose, polyhydroxyethyl methacrylate (PHEMA), polyvinyl alcohol (PVA), polyvinyl pyrrolidone (PVP), polyethylene oxide (PEO), or polyacrylamide (PA), carrageenan, sodium alginate, polycarbophil, polyacrylic acid, tragacanth, methyl cellulose, pectin, natural gums, xanthan gum, guar gum, karaya gum, hypromellose, magnesium stearate, microcrystalline cellulose, and colloidal silicon dioxide. Suitable antioxidants are vitamin A, carotenoids, vitamin C, vitamin E, selenium, flavonoids, polyphenols, lycopene, lutein, lignan, coenzyme Q10 ("CoQIO") and glutathione.

The unit dosage forms, especially those in sachet form, can also include various nutrients including macronutrients.

The unit dosage forms can be administered orally, e.g. as a tablet, capsule, or pellet containing a predetermined amount, or as a powder or granules containing a predetermined concentration or a gel, paste, solution, suspension, emulsion, syrup, bolus, electuary, or slurry, in an aqueous or non-aqueous liquid, containing a predetermined concentration. Orally administered compositions can include binders, lubricants, inert diluents, flavouring agents, and humectants. Orally administered compositions such as tablets can optionally be coated and can be formulated so as to provide sustained, delayed or controlled release of the mixture therein.

The unit dosage forms can also be administered by rectal suppository, aerosol tube, naso-gastric tube or direct infusion into the GI tract or stomach.

The unit dosage forms can also include therapeutic agents such as antiviral agents, antibiotics, probiotics, analgesics, and anti-inflammatory agents. The synthetic composition in unit dosage form may be a pharmaceutical composition or a nutritional supplement.

### Administration dosing

For reducing or preventing symptoms of visceral pain in a patient, the amount of HMO(s), required to be administered to the patient, will vary depending upon factors such as the risk and severity of the disease, the age of the patient, the form of the composition, and other medications being administered to the patient. However, the required amount can be readily set by a medical practitioner and would generally be in the range from about 200 mg to about 20 g per day, in certain embodiments from about 1 g to about 15 g per day, from about 3 g to about 10 g per day, in certain embodiments from about 3 g to about 7.5 g per day. An appropriate dose can be determined based on several factors, including, for example, body weight and/or condition, the severity of the condition, being treated or prevented, other ailments and/or diseases, the incidence and/or severity of side effects and the manner of administration. Appropriate dose ranges may be determined by methods known to those skilled in the art. During an initial treatment phase, the dosing can be higher or lower depending upon the need to boost bifidobacteria abundance or initial tolerance to HMOs. During a maintenance phase, the dosing can be set for chronic long term use.

The duration of the HMO administration will vary depending upon factors such as the risk and severity of the medical condition, age, the form of the composition, the dose and other medications being administered. However, the duration can be readily set by a medical practitioner. Generally, a duration of at least a week will be required to sufficiently to impact symptoms. For example, the duration may be for 1 to 3 months. The administration can continue chronically for an indefinite period.

### EXAMPLES

Examples are to illustrate non-limiting embodiments the invention.

### Example 1- Human trial

A total of 60 male and female IBS patients are recruited to participate in the study. After a screening visit and run-in period of 1-2 weeks, the patients are selected. The patients are randomized into three groups, each of 20 patients, with two groups consuming the treatment product and one group the placebo product for 4 weeks. The treatment product contains either 5 or 10 grams of a combination of 2'-FL and LNnT in a 4:1 ratio, while the placebo product contains 5 grams of glucose. Both products are in powder form in a unit dosage container.

The patients are eligible to participate if they are at an age between 18-60 years, fulfil definition of IBS-D, IBS-C or IBS-A/M according to the Rome IV criteria for IBS and have a global IBS-SSS score of >174 during the 2 weeks run-in period. All recruited patients are able and willing to understand and comply with the study procedures. Patients are excluded if: they have any known gastrointestinal disease(s) that may cause symptoms or interfere with the trial outcome, in particular lactose intolerance and coeliac disease; they have participated in a clinical study one month prior to screening visit; they have abnormal results in the screening tests which are clinically relevant for study participation; they are suffering for a severe disease such as malignancy, diabetes, severe coronary disease, kidney disease, neurological disease, or severe psychiatric disease or any condition which can confound the results of the study; used highly dosed probiotic supplements (yoghurt allowed) for 1 months prior to the study; consumed antibiotic drugs 1 months prior to the study; consumed on a regular basis any medication that might interfere with symptom evaluation 2 weeks prior to the study; diagnosed with and treated for IBS for more than 10 years; and pregnant or lactating.

At the screening visit, clinical and medical history and concomitant medication is registered. IBS diagnostic criteria will be assessed and part 2 of the IBS-SSS questionnaire will be completed.

A faecal sample kit is distributed together with the Bristol Stool Form Scale (BSFS) and Bowel Movement Diary (BMD) to be filled in during the 7 days just prior to visit 2. Patients will be asked to register their diet 3 days just prior to visit 2, and will be reminded not to change their usual diet during the study.

At the second visit, eligibility criteria are checked and eligible subjects are randomised to the three arms in the trial. A physical examination is done and a number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Based on clinical symptoms and data from questionnaires, patients are characterised into one of the three following groups; diarrhoea predominant (IBS-D), constipation predominant (IBS-C) or alternating/mixed (IBS-A/M). This enables allocation of patients from each subgroup into the intervention groups. When allocated to the groups patients are provided with either treatment or placebo products. Sigmoidoscopy is performed and mucosal biopsies and faecal aspirates taken. Patients are asked about any adverse events and any changes in their usual medication. The BSFS and BMD is collected and new forms, to be filled in daily during the intervention period, are distributed. Faecal samples are collected and equipment for new samples are distributed. Blood samples are collected for routine clinical chemistry and haematology and biomarker analysis and a saliva sample is collected to analyse FUT2 secretor status. Diet records are collected, and patients are asked to register their diet for 3 days just prior to visit 3. Patients are reminded not to change their usual diet during the study.

At the third visit, a physical examination is performed and a number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Remaining study products and compliance diaries are collected to check compliance. Blood samples are collected for routine clinical chemistry and haematology and biomarker analysis, and sigmoidoscopy is performed and mucosal biopsies and faecal aspirates taken. Patients are asked about any adverse events and any changes in their usual medication. Faecal samples are collected and equipment for collecting new samples distributed. The BSFS and BMD is collected and new forms, to be filled in during the 7 days just prior to visit 4, are distributed. Diet records are collected, and patients are reminded not to change their usual diet during the study.

The treatment period lasts 4 weeks, the patients are administered 5 or 10 g of mix of 2'-FL + LNnT or 5 g of glucose daily. Patients are instructed to consume the products in the morning with breakfast. Compliance is monitored through the interactive internet enabled system. At the end of the study, each patient has an exit visit with the medical team. Faecal samples and blood samples are collected and analysed as before. A number of questionnaires (GSRS-IBS, IBS-SSS, HADS, NRS-11, VSI, IBS-QOL and PHQ-15 scales) are answered. Questionnaires are filled in electronically. Those who are unable or unwilling to use the electronic system fill out the questionnaires on paper. Patients are asked about any adverse events and any changes in their usual medication or diet, and the BSFS and BMD is collected.

To assess the microbiota profile, DNA is extracted from faecal samples using a 96-well PowerSoil DNA Isolation Kit (MO-BIO). A minimum of one sample-well per plate is kept empty to serve as a negative control during PCR. PCR is done with the forward primer S-D-Bact-0341-b-S-17 and reverse primer S-D-Bact-0785-a-A-21 with IIIumina adapters attached (Klindworth et al. Nucleic Acids Res. 41, e1 (2013)). These are universal bacterial 16S rDNA primers, which target the V3-V4 region. Following PCR program is used: 98°C for 30 sec, 25x (98°C for 10 s, 55°C for 20 s, 72 °C for 20 s), 72 °C for 5 min. Amplification is verified by running the products on a 1 % agarose gel. Barcodes are added in a nested PCR using the Nextera Index Kit V2 (illumina) with the following PCR program: 98°C for 30 sec, 8x (98°C for 10 s, 55°C for 20 s, 72 °C for 20 s), 72 °C for 5 min. Attachment of primers is verified by running the products on a 1 % agarose gel. Products from the nested PCR are normalized using the SequalPrep Normalization Plate Kit and pooled. Pooled libraries are concentrated by evaporation and the DNA concentration of pooled libraries is measured on a Qubit fluorometer using the Qubit High Sensitivity Assay Kit (Thermo Fisher Scientific). Sequencing is done on a MiSeq desktop sequencer using the MiSeq Reagent Kit V3 (illumina) for 2 x 300 bp paired-end sequencing. The 64-bit version of USEARCH is used for bioinformatical analysis of the sequence data.

The results show that oral ingestion of HMOs modulate the intestinal microbiota, and specifically stimulate the abundance of bifidobacteria. The blood biomarker analysis indicates that the treatment patients have reduced levels of inflammatory markers, and the biopsy analysis reveals reduction in activated mast cell meaning stabilization of mast cells. Reduction in visceral pain and an improvement in bowel movement are reported by treatment patients as compared to the placebo group. Collectively, HMOs are able to increase bifidobacteria and stabilize mast cells, and hereby contribute to improvement in visceral pain in IBS patients.

### Example 2 - Nutritional composition

A ready to feed nutritional composition is prepared from water, maltodextrin, corn syrup, sugar, milk protein concentrate, vegetable oil (canola, high oleic sunflower and corn), soy protein isolate, acacia gum, flavours, HMSs/HMOs, potassium citrate, magnesium phosphate, cellulose gel and gum, calcium carbonate, sodium ascorbate, soy lecithin, choline bitartrate, calcium phosphate, alpha-tocopheryl acetate, ascorbic acid, carrageenan gum, ferric pyrophosphate, flavours, sweeteners (Stevia), vitamin A palmitate, niacinamide, vitamin D3, calcium pantothenate, manganese sulphate, copper sulphate, pyridoxine hydrochloride, thiamine hydrochloride, beta carotene, riboflavin, chromium chloride, folic acid, biotin, potassium iodide, phytonadione, sodium selenite, sodium molybdate, vitamin B12.

The composition provides a nutritional supplement which is a good source of protein, low in fat, vitamins, minerals and antioxidants, and meets FODMAP criteria. Further, the composition contains HMSs and/or HMOs which are able to promote the growth of beneficial intestinal bacteria and modulate chronic inflammation.

### Example 3 - Capsule composition

A capsule is prepared by filling about 1 g of HMS/HMO into a 000 gelatine capsule using a filing machine. The capsules are then closed. The HMS/HMO are in free flowing, powder form.

### Example 4 - Mucosal barrier function

2'-FL and LNnT are tested with respect to their ability to induce MUC2, TFF3, EIMβ, CHST5, and GAL3ST2 expression in the human LS174T cell culture model of goblet cells. The human LS174T cell line is obtained from the American Type Culture Collection (ATCC). LS174T cells are maintained in minimum essential medium (MEM) supplemented according to instructions at 37 °C in 5% CO₂. 2'-FL and LNnT are dissolved in cell culture grade water to the required concentration. The LS174T cells are treated with the HMO solution containing 0 or 5 mg HMO/ml.

The LS174T cells are collected and suspended in Trizol reagent and total RNA is isolated using an RNA analysis kit (Qiagen) according to the manufacturer's instructions and the RNA isolates are quantified using Nanodrop analysis (Thermo Fisher Scientific). RNA isolates are reverse transcribed using a high capacity cDNA Reverse Transcription Kit (Applied Biosystems) to create cDNA, which is then used to assess gene expression via quantitative RT-PCR.

For the quantitative RT-PCR, specific TaqMAN gene expression assays are obtained from Applied Biosystems, which include expression assays for MUC2, TFF3, CHST5 and GAL3ST2. Quantitative real-time PCR is performed using TaqMAN PCR Master Mix (Applied Biosystems). Reactions are run in duplicates in a 384-well plate using an Applied Biosystems 7900HT Fast Real-Time PCR System. The results are analysed using SDS 2.3 software and calculated by delta delta Ct method. All samples are normalized to Gus-β expression and fold induction is calculated over untreated controls. Gene expression is expressed as fold increase compared to HMO-free control cells. The experiment is repeated three times.

The results indicate that treatment with 2'-FL and LNnT increases the expression of the MUC2 and TFF3 genes compared to control cultures. Increased expression of goblet cell genes is specific and not universal, as evidenced by the minimal induction or lack of induction of CHST5 and GAL3ST2, respectively. MUC2 and TFF3 are key components of the mucosal barrier and improve mucosal barrier function.

## Claims

1. One or more human milk oligosaccharides (HMOs) for use in the prophylaxis or treatment of mast cell mediated visceral hypersensitivity and/or mast cell mediated visceral pain in a human.

2. A synthetic composition for use in the prophylaxis or treatment of mast cell mediated visceral hypersensitivity and/or mast cell mediated visceral pain in a human, the composition comprising an effective amount of a human milk oligosaccharide.

3. One or more HMOs for the use according to claim 1 or the synthetic composition for the use according to claim 2, wherein the human has one or more of bacterial overgrowth, dysbiosis and an impaired mucosal barrier.

4. One or more HMOs or the synthetic composition for the use according to any one of claims 1 to 3, wherein the human is an IBS patient.

5. One or more HMOs for the use according to any one of claims 1 and 3 to 4 effective to increase (i) the abundance, particularly the relative abundance, of bifidobacteria, and/or (ii) improve the gut barrier function of the human.

6. The synthetic composition for the use according to any one of claims 2 to 4, comprising an amount of the HMO effective to (i) increase the abundance, particularly the relative abundance, of bifidobacteria, in the gastrointestinal tract of the human, and/or (ii) improve the gut barrier function of the human.

7. One or more HMOs for the use according to claim 5 or the synthetic composition for the use according to claim 6, wherein the bifidobacteria, in the initial treatment period, are those of the phylogenetic *Bifidobacterium adolescentis* group and, after about 14 days of treatment, are *Bifidobacterium longum* and/or *Bifidobacterium bifidum.*

8. One or more HMOs or the synthetic composition for the use according to claim 7, wherein the bifidobacterium of the phylogenetic *Bifidobacterium adolescentis* group is *Bifidobacterium pseudocatenulatum* and/or *Bifidobacterium adolescentis.*

9. One or more HMOs for the use according to any one of claims 1, 3 to 5 and 7 to 8, wherein the HMO is a fucosylated or a non-fucosylated neutral HMO.

10. One or more HMOs for the use according to any one of claims 1, 3 to 5 and 7 to 8, wherein the HMO is 2'-FL, 3-FL, DFL, LNnT, LNT, 3'-SL, 6'-SL or LNFP-I, or a mixture thereof.

11. One or more HMOs for the use according to claim 10, wherein the one or more HMOs is a mixture comprising or essentially consisting of 2'-FL and LNnT and/or LNT.

12. The synthetic composition for the use according to any one of claims 2 to 4 and 6 to 8, wherein the HMO is a fucosylated or a non-fucosylated neutral HMO.

13. The synthetic composition for the use according any one of claims 2 to 4 and 6 to 8, wherein the HMO is 2'-FL, 3-FL, DFL, LNnT, LNT, 3'-SL, 6'-SL or LNFP-I, or a mixture thereof.

14. The synthetic composition for the use according to claim 13, wherein the HMO is a mixture comprising or consisting essentially of 2'-FL and LNnT and/or LNT.

15. The synthetic composition for the use according to claim 14, wherein the amount of the 2'-FL and LNnT is at least 3 g and wherein the 2'-FL/LNnT mass ratio is 2:1 to 5:1.

## Patentansprüche

1. Ein oder mehrere humane Milch-Oligosaccharide (HMOs) zur Verwendung bei der Prophylaxe oder Behandlung von mastzellenvermittelter viszeraler Hypersensibilität und/oder mastzellenvermitteltem viszeralem Schmerz in einem Menschen.

2. Eine synthetische Zusammensetzung zur Verwendung bei der Prophylaxe oder Behandlung von mastzellenvermittelter viszeraler Hypersensibilität und/oder mastzellenvermitteltem viszeralem Schmerz in einem Menschen, wobei die Zusammensetzung eine wirksame Menge von einem humanen Milch-Oligosaccharid umfasst.

3. Ein oder mehrere HMOs zur Verwendung nach Anspruch 1 oder die synthetische Zusammensetzung zur Verwendung nach Anspruch 2, wobei der Mensch eines oder mehrere von bakterieller Überbesiedelung, Dysbiose und einer gestörten Schleimhautbarriere aufweist.

4. Ein oder mehrere HMOs oder die synthetische Zusammensetzung zur Verwendung nach einem der Ansprüche 1 bis 3, wobei der Mensch ein RDS-Patient (Reizdarmsyndrom-Patient) ist.

5. Ein oder mehrere HMOs zur Verwendung nach einem der Ansprüche 1 und 3 bis 4, die wirksam sind, um (i) die Abundanz, insbesondere die relative Abundanz, von Bifidobakterien zu erhöhen, und/oder (ii) die Darmbarrierefunktion des Menschen zu verbessern.

6. Die synthetische Zusammensetzung zur Verwendung nach einem der Ansprüche 2 bis 4, die eine Menge von dem HMO umfasst, die wirksam ist, um (i) die Abundanz, insbesondere die relative Abundanz, von Bifidobakterien in dem Gastrointestinaltrakt des Menschen zu erhöhen, und/oder (ii) die Darmbarrierefunktion des Menschen zu verbessern.

7. Ein oder mehrere HMOs zur Verwendung nach Anspruch 5 oder die synthetische Zusammensetzung zur Verwendung nach Anspruch 6, wobei es sich bei den Bifidobakterien, in dem anfänglichen Behandlungszeitraum, um solche der phylogenetischen Gruppe *Bifidobacterium adolescentis* und, nach etwa 14 Tagen Behandlung, um *Bifidobacterium longum* und/oder *Bifidobacterium bifidum* handelt.

8. Ein oder mehrere HMOs oder die synthetische Zusammensetzung zur Verwendung nach Anspruch 7, wobei es sich bei dem Bifidobakterium der phylogenetischen Gruppe *Bifidobacterium adolescentis* um *Bifidobacterium pseudocatenulatum* und/oder *Bifidobacterium adolescentis* handelt.

9. Ein oder mehrere HMOs zur Verwendung nach einem der Ansprüche 1, 3 bis 5 und 7 bis 8, wobei das HMO ein fucosyliertes oder ein nicht fucosyliertes neutrales HMO ist.

10. Ein oder mehrere HMOs zur Verwendung nach einem der Ansprüche 1, 3 bis 5 und 7 bis 8, wobei das HMO 2'-FL, 3-FL, DFL, LNnT, LNT, 3'-SL, 6'-SL oder LNFP-I oder ein Gemisch davon ist.

11. Ein oder mehrere HMOs zur Verwendung nach Anspruch 10, wobei das eine oder die mehreren HMOs ein Gemisch sind, das 2'-FL und LNnT und/oder LNT umfasst oder im Wesentlichen daraus besteht.

12. Die synthetische Zusammensetzung zur Verwendung nach einem der Ansprüche 2 bis 4 und 6 bis 8, wobei das HMO ein fucosyliertes oder ein nicht fucosyliertes neutrales HMO ist.

13. Die synthetische Zusammensetzung zur Verwendung nach einem der Ansprüche 2 bis 4 und 6 bis 8, wobei das HMO 2'-FL, 3-FL, DFL, LNnT, LNT, 3'-SL, 6'-SL oder LNFP-I oder ein Gemisch davon ist.

14. Die synthetische Zusammensetzung zur Verwendung nach Anspruch 13, wobei das HMO ein Gemisch ist, das 2'-FL und LNnT und/oder LNT umfasst oder im Wesentlichen daraus besteht.

15. Die synthetische Zusammensetzung zur Verwendung nach Anspruch 14, wobei die Menge von dem 2'-FL und dem LNnT mindestens 3 g beträgt, und wobei das 2'-FL/LNnT-Masseverhältnis 2:1 bis 5:1 beträgt.

## Revendications

1. Un ou plusieurs oligosaccharides de lait humain (OLH) destiné à être utilisé dans la prophylaxie ou le traitement de l'hypersensibilité viscérale induite par des mastocytes et/ou dans la douleur viscérale induite par des mastocytes chez l'humain.

2. Une composition synthétique destinée à être utilisée dans la prophylaxie ou le traitement de l'hypersensibilité viscérale induite par des mastocytes et/ou dans la douleur viscérale induite par des mastocytes chez l'humain, la composition comprenant une quantité effective d'un oligosaccharide de lait humain.

3. Un ou plusieurs OLH destinés à être utilisés selon la revendication 1 ou la composition synthétique destinée à être utilisée selon la revendication 2, dans lequel/lesquels l'humain présente l'une ou plusieurs parmi une prolifération bactérienne, une dysbiose et une barrière muqueuse déficiente.

4. Un ou plusieurs OLH ou la composition synthétique destinés à être utilisée selon l'une quelconque des revendications 1 à 3, dans lesquels l'humain est un patient souffrant du SCI (syndrome du colon irritable).

5. Un ou plusieurs OLH destinés à être utilisés selon l'une quelconque des revendications 1 et 3 à 4, permettant d'augmenter (i) l'abondance, en particulier l'abondance relative, de bifidobactéries, et/ou (ii) d'améliorer la fonction barrière intestinale de l'humain.

6. La composition synthétique destinée à être utilisée selon l'une quelconque des revendications 2 à 4, comprenant une quantité d'OLH permettant (i) d'augmenter l'abondance, en particulier l'abondance relative, de bifidobactéries dans le tube digestif de l'humain, et/ou (ii) d'améliorer la fonction de barrière intestinale de l'humain.

7. Un ou plusieurs OLH destinés à être utilisés selon la revendication 5 ou la composition synthétique destinée à être utilisée selon la revendication 6, dans lesquels les bifidobactéries, dans la période initiale de traitement, sont celles du groupe phylogénétique Bifidobacterium adolescentis et, après environ 14 jours de traitement, sont Bifidobacterium longum et/ou Bifidobacterium bifidum.

8. Un ou plusieurs OLH ou la composition synthétique destinés à être utilisés selon la revendication 7, dans lesquels la bifidobactérie du groupe phylogénétique Bifidobacterium adolescentis est Bifidobacterium pseudocatenulatum et/ou Bifidobacterium adolescentis.

9. Un ou plusieurs OLH destinés à être utilisés selon l'une quelconque des revendications 1, 3 à 5 et 7 à 8, dans lesquels l'OLH est un OLH fucosylaté ou neutre non fucosylaté.

10. Un ou plusieurs OLH destinés à être utilisés selon l'une quelconque des revendications 1, 3 à 5 et 7 à 8, dans lesquels l'OLH est le 2'-FL, le 3-FL, le DFL, le LNnT, le 3'-SL, le 6'-SL ou le LNFP-I, ou un mélange de ceux-ci.

11. Un ou plusieurs OLH destinés à être utilisés selon la revendication 10, dans lesquels les un ou plusieurs OLH sont un mélange comprenant ou consistant essentiellement dans 2'-FL et du LNnT et/ou du LNT.

12. La composition synthétique destinée à être utilisée selon l'une quelconque des revendications 2 à 4 et 6 à 8, dans laquelle l'OLH est un OLH fucosylaté ou neutre non fucosylaté.

13. La ccomposition synthétique destinée à être utilisée selon l'une quelconque des revendications 2 à 4 et 6 à 8, dans laquelle l'OLH est le 2'-FL, le 3-FL, le DFL, le LNnT, le 3'-SL, le 6'-SL ou le LNFP-I, ou un mélange de ceux-ci.

14. La composition synthétique destinée à être utilisée selon la revendication 13, dans laquelle l'OLH est un mélange comprenant ou consistant essentiellement en du 2'-FL et du LNnT et/ou du LNT.

15. La composition synthétique destinée à être utilisée selon la revendication 14, dans laquelle la quantité du 2'-FL et du LNnT est d'au moins 3 g et dans laquelle le rapport massique 2'-FL/LNnT est compris entre 2:1 et 5:1.
